(19) Europäisches Patentamt
European Patent Office
Office européen des brevets

(11) **EP 2 639 723 A1**

(12) **EUROPEAN PATENT APPLICATION**

(43) Date of publication:
**18.09.2013 Bulletin 2013/38**

(51) Int Cl.:
***G06F 19/00*** *(2011.01)*

(21) Application number: **13168616.4**

(22) Date of filing: **20.10.2004**

(84) Designated Contracting States:
**AT BE BG CH CY CZ DE DK EE ES FI FR GB GR HU IE IT LI LU MC NL PL PT RO SE SI SK TR**

(30) Priority: **20.10.2003 US 512908 P**

(62) Document number(s) of the earlier application(s) in accordance with Art. 76 EPC:
**04795768.3 / 1 683 038**

(71) Applicant: **Zoll Medical Corporation**
**Chelmsford**
**Massachusetts 01824-4105 (US)**

(72) Inventors:
• **De Zwart, Aga**
**Nelson, British Columbia V1L 4L3 (CA)**

• **Kurucz, Frank**
**Loveland, CO Colorado 80537 (US)**
• **Cohen, David G**
**Boulder, CO Colorado 80301 (US)**
• **Freeman, Gary A**
**Newton Center, MA Massachusetts 02159 (US)**

(74) Representative: **Potter Clarkson LLP**
**The Belgrave Centre**
**Talbot Street**
**Nottingham**
**NG1 5GG (GB)**

<u>Remarks:</u>
This application was filed on 21-05-2013 as a divisional application to the application mentioned under INID code 62.

(54) **Portable medical information device with dynamically configurable user interface**

(57) A portable electronic device for recording medical data, including a display, electronics for displaying a user interface on the display and for responding to user inputs entered on the device, and electronics for determining the environment in which the device is being used, wherein the user interface is varied in accordance with the determined environment.

Figure 2

## Description

Cross-Reference to Related Application

This application claims priority to U.S. Application Serial No. 60/512,908, filed on October 20, 2003.

Technical Field

This invention relates to portable medical information devices.

Background

Emergency medical care delivered to a patient occurs primarily in one of two settings: in hospitals by nurses and physicians, and in the field by trained emergency service providers, typically in the form of police officers, emergency medical technicians (EMTs), fire departments, paramedics or physicians in some cases. In the best medical systems, programs are put into place to assess current levels of care and to provide continuous quality-of-care improvements. Common measures of system effectiveness are endpoints such as survival, in the case of cardiac arrest, or improvement in health, in the case of non-terminal events. Additional interim measures are also important, however in determining areas for improvement in care; these data include response time and protocol adherence. While electronic patient charting software is now available, it is not uncommon to still see paper run reports being generated by emergency health care providers to record a patient's relevant personal information as well as the specifics of the vital signs of the patient and treatments delivered to the patient. The so-called run report or patient chart (RRPC) can subsequently be used by medical supervisory persons such as the Medical Director to determine statistical summaries of medical care performance. A common reporting format for care and outcomes, particularly in the pre-hospital setting, is the Utstein Style format as promulgated by the American Heart Association and other organizations. It is often the case that computers are used to enter data from paper run reports, the subsequent digital data then being processed to determine the aforementioned outcome and quality of care statistics as well as paper and electronic reports. These computer-based analyses and reporting programs typically have user interfaces unique to that product, and the medical supervisory personnel are often burdened with the difficulty of learning new software functionality when new versions or products are available, and at times required to maintain skills in multiple complex analysis programs.

Portable computing devices providing electronic versions of RRPC have been available for a number of years on laptops and other portable computing devices with screens large enough to display a significant portion of the information necessary for their relatively efficient use. The bulk, price and weight of these computing devices as well as the awkwardness of handling the large devices has precluded, however, widespread acceptance of electronic patient records. More recently, RRPCs have been implemented on PDAs, providing a more portable and convenient device for the health care provider, but as a consequence of the very small size of the display, resulting in devices less convenient with which to interact.

Summary

In general the invention features a user interface for portable electronic devices used for recording medical data (e.g., recording events on an electronic RRPC). The user interface is dynamically reconfigured in accordance with the particular environment of use. The invention provides a more efficient process by which medical personnel may enter data into and interact with an electronic medical record. The invention can be used on a wide variety of portable electronic device, including personal digital assistants (PDAs), Tablet PCs, and laptop computers. It has applicability to any device that is used for electronically recording medical information such as treatments delivered to a patient, actual protocols followed during a medical procedure, and medical events and data arising from the medical or physiological condition of the patient.

The invention features a portable electronic device for recording medical data, comprising a display, electronics for displaying a user interface on the display and for responding to user inputs entered on the device, and electronics for determining the environment in which the device is being used, wherein the user interface is varied in accordance with the determined environment.

In preferred implementations of the invention may incorporate one or more of the following: The display may be touch sensitive, and user inputs may be made by touching selected portions of the display. The user interface may comprise buttons, and the choice of buttons displayed at a given time may be varied depending on the environment in which the portable device is used. The user interface may comprise buttons and the position of the buttons at a given time may be varied depending on the environment in which the portable device is used. The user interface may present lists of items from which the user makes a selection, and a list presented at a given time may contain a subset of items, wherein the content of the subset may be varied in accordance with the environment in which the portable device is used. The user interface may be varied depending on detection of whether the user is holding the portable device with the left or the right hand. The device may comprise a sensor and related electronics for automatically detecting whether the portable device is being held along its right or left edge. The device may further comprise electronics for communication with external devices, and the user interface may be varied depending on the type of external device with which the portable device is communicating. The device may further comprise electronics for communication with an external device, wherein the external device operates in a plurality of modes, and the mode in which the external device is operating is communicated to the portable device, and the user interface may be varied depending on the mode in which the external device is operating. The device may further comprise electronics for communication with an external device, and the user interface may be varied in accordance with whether the portable device is in the vicinity of an external device (e.g., a transmitter for informing the portable device that it is in the vicinity of the emergency room of a hospital, or a transmitter for informing the portable device that it is in an ambulance). The user interface may be varied depending on measurements made by the external device and communicated to the portable device. Communication with an external device may be in the form of wireless communication. The user interface may be varied depending on the medical interventions entered by the user. The user interface may be varied depending on the frequency with which actions have been taken in the past by the user. The user interface may be varied to reduce the number of steps necessary to select actions frequently taken in the past by the user (e.g., a frequently selected action may appear at the top of a list in the user interface). The user interface may be varied depending on the frequency with which actions have been taken in the past by the user. The user interface may be varied to reduce the number of steps necessary to select actions frequently taken in the past by the user. A frequently selected action may appear at the top of a list in the user interface. The user interface may be varied based on a predictive algorithm. The predictive algorithm may comprise one or more statistical learning methods. The predictive algorithm may comprise a ranked-frequency listing of recent choices from a list.

Among the many advantages of the invention (some of which may be achieved only in some of its various aspects and implementations) are that the user interface is easier to use, and makes possible, for the first time, the successful use of small area graphical user interfaces (e.g., as typically found on PDAs) as RRPC devices.

Brief Description of the Drawings

FIG. 1 is a screen shot of a user interface according to one implementation of the invention.
FIG. 2 is a depiction of a PDA according to one implementation of the incoporating software using one or more implementations of the invention.
FIG. 3 is a block diagram of one implementation of the invention.

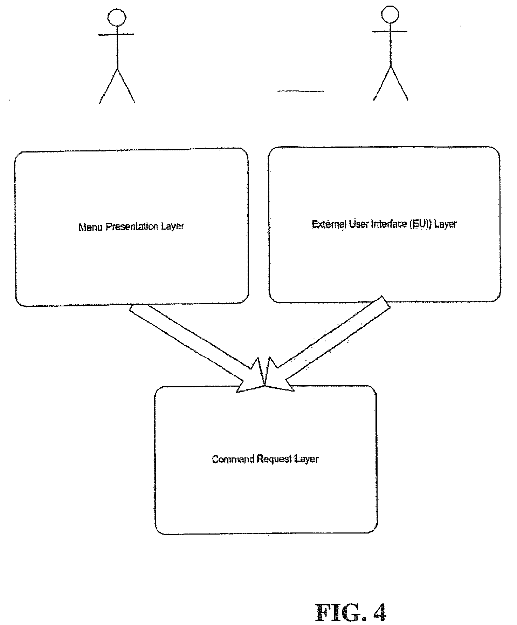

FIG. 4 is a flow chart of the user interface functionality of an implementation of the invention.
FIG. 5 is a flow chart of the user interface functionality of an implementation of the invention.
FIG. 6 is a block diagram of the medical response system utilizing the invention.

Detailed Description

There are a great many possible implementations of the invention, too many to describe herein. Some possible implementations that are presently preferred are described below. It cannot be emphasized too strongly, however, that these are descriptions of implementations of the invention, and not descriptions of the invention, which is not limited to the detailed implementations described in this section but is described in broader terms in the claims.

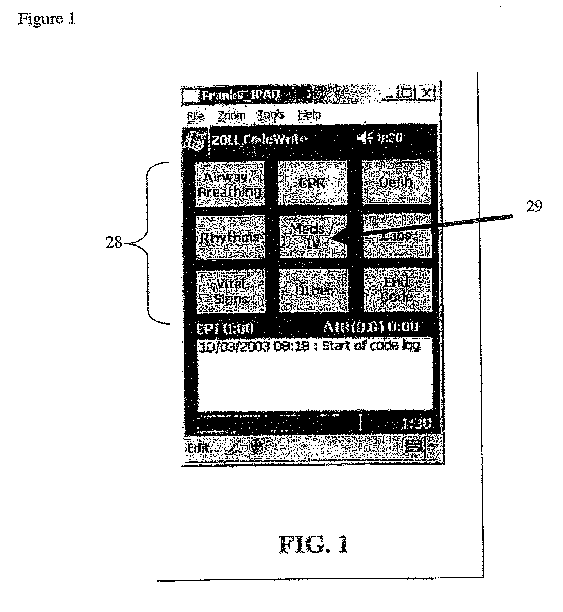

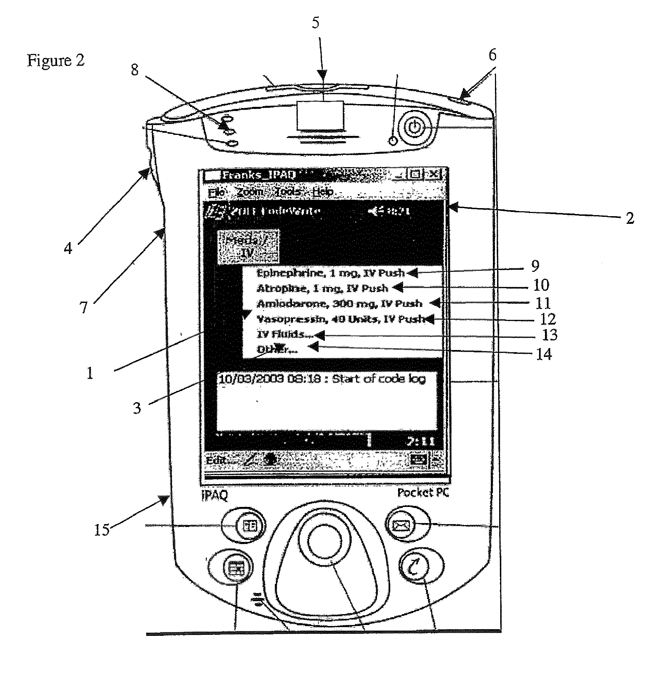

Referring to FIGS. 1 and 2, in some implementations, a portion of an extended list 1 of medical interventions is displayed by a graphical user interface 2, the extent and order of the list portion determined by the prior history of medical interventions entered by that particular user on that device. Through a user input means such as touch screen 3 or jog wheel 4, the user is able to select one or more of the items from the displayed list 1 portion for storage in the RRPC device 15. Similar preferences can be automatically stored for a particular user such as repetitive or complex user interaction sequences.

One implementation of the user interface includes the screen displays shown in FIGS. 1-2. Each of the nine rectangular areas 28 of the screen shown in FIG. 1 is a touch-sensitive button by which the user can initiate certain actions. The result of touching the center button, Meds/IV 29, is shown in FIG. 2. A list of drugs is presented to allow the user to select the drug that has been administered. The user interface has been simplified by keeping the number of clicks to a minimum for common documentation. Thus, to enter into the log that a particular drug has been used, one click is required on the Meds/IV button 29, and a second click is required to choose from among the drugs appearing in the list 1. In some cases, this documentation is detailed with answers to multiple levels of questions. For example, if the drug atropine 10 is given, a further screen (not shown) appears asking for dosage, routes, and other related questions.

The user interface is designed to dynamically adapt to the user's prior history of usage. For example, if the user routinely selects 2mg intravenous, then this answer will appear at the top of the list in the user interface, to make it easier for the user to make this typical selection. A database of answers for a particular user is maintained, so that over time the interface can predict with reasonable accuracy the most likely choice that a user will make in response to a question. For example, in FIG. 2 the drug selections, Epinephrine, 1 mg and Atropine, 1 mg appear at the top of the list by methods that will be descibed in more detail below. Although not shown in the figures, the user interface can present the user with single click selections for common procedures.

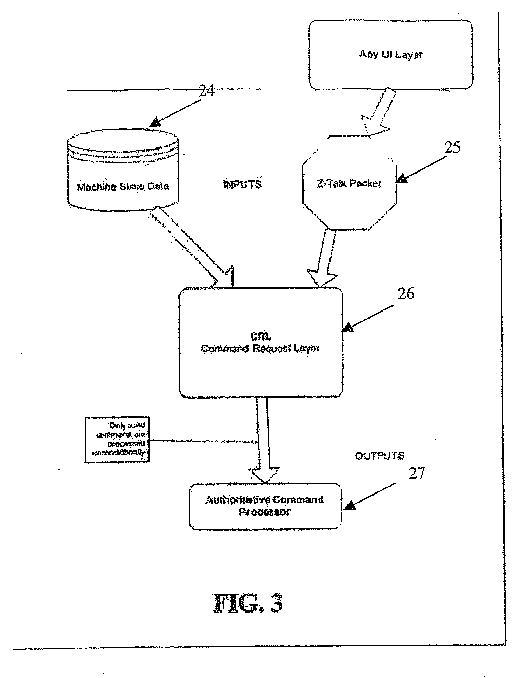

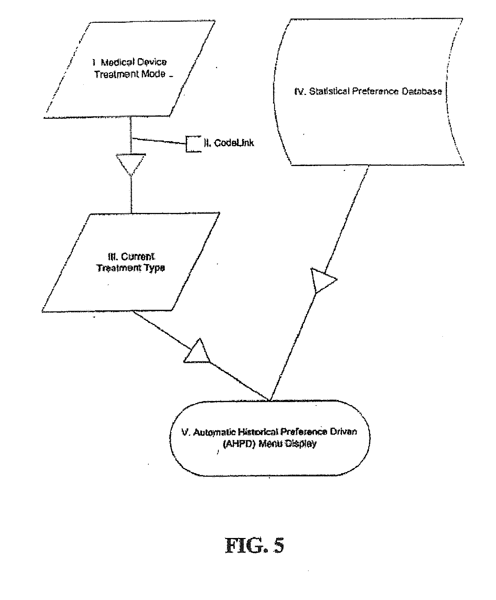

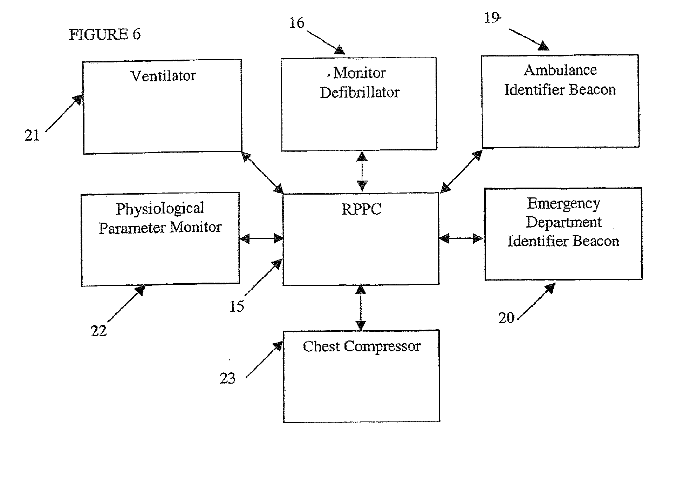

In other implementations, the RRPC device 15 is able to communicate with one or more diagnostic or therapeutic medical devices in the vicinity of the emergency procedure. The communication may be accomplished through such well-known wireless means as optical methods such as infra-red embodied in the IrDA 5 standard, or RF communication such as Bluetooth or 802.11 standards, or other similar methods with built-in wireless electronics and antenna 6 on the PDA. Based on the communications from the devices in the vicinity of the emergency procedure, the RRPC device 15 is able to ascertain the type of emergency procedure in progress and automatically configure the user interface to be in accordance with the procedure in session. More specifically, a transthoracic pacemaker/defibrillator 16 such as the M-Series manufactured by ZOLL Medical of Chelmsford MA as shown in the block diagram of FIG 6 and data diagram of FIG. 5, if set to PACE mode of operation, would communicate this state information 17 to the RRPC device 15, which would then configure the list portion so that the medical interventions appropriate for pacing would be displayed. In a similar fashion, different list portions 1 would be configured depending on whether the pacemaker/defibrillator 16 was in Monitoring mode or Defibrillation mode. The RRPC device 15 may incorporate specific patient physiologic information such as ECG, pulse oximetry or other parameter from a diagnostic or therapeutic medical device 18 in the vicinity of the emergency procedure to dynamically reconfigure the user interface. For instance, measurements can be taken of the ST segment of the ECG on a continual basis, and if the value exceeds a predetermined threshold, the list portion may be altered to include treatments related to myocardial infarction. FIG. 3 is a block diagram of the processing of communication between the RRPC device 15 and the Defibrillator or other therapeutic medical device 18. Communication can be via infrared or Bluetooth (or a cable) communication medium that can be either real-time or post-processed. Packets (e.g., Z-Talk packets) 25 are transmitted from the user interface to the Command Request Layer (CRL) 26, which is also in receipt of machine state data. The CRL 26 issues outputs to the Authoritative Command Processor 27. Through the use of Bluetooth technology, it is now possible to sense physical proximity to other devices. If the user is using Bluetooth in both the data capture device (PDA or other portable device) and the receiving station (desktop on local area network) then the software can automatically sense proximity of these two locations, and begin formatting records for transfer and automatically transferring them if configured in this manner. This can be accomplished using Bluetooth "discovery" features defined by the Bluetooth special interest group standards.

The RRPC device 15 may be capable of determining whether the operator is holding the device in their left or right hand, and thus using their left or right thumb, for example, to operate the device. This determination could be made using touch or pressure sensors 7 along the edges of the device as shown in FIG. 2, or by questioning the user either by text prompts on the touchscreen 3 or by audio means from the speaker output 8 . Based on this determination, the touchscreen input fields 9-14 for the list portion 1 will be placed on the left or right side of the screen 2 in order to make one-handed operation of the device possible. In preferred implementations, the user is asked (via a user preference) which hand is being used to hold the device (left or right). Alternatively, the device could sense whether the user is using the right or left hand (based on whether the right or left edge of the device is being grasped). Based on the answer as to which hand is holding the device, the user interface alters the orientation of the menus. On the main documentation screen, there are a series of large buttons 28. When a button is tapped, it moves (via animation) up and to the left if the device is held in the right hand. This causes the menu to appear below and to the right of the button, and also causes menus to originate from the same coordinate on the screen. In turn, this gives the user holding the device in his right hand the best angle for accurate menu selection. For users holding the device in their left hand, the user interface instead animates the button to the top right after being pressed, and the menu descends to the left and down, improving the ergonomics (and ultimately recording accuracy) for that user.

Alternatively, handwriting recognition software can determine the dominant hand of the user (left vs. right) such that this setting can be automatically applied. Determination of handedness of the user can be accomplished by such methods as average stroke angle computed across a series of letters; if the stroke angle is greater than preferably 5 degrees clockwise from vertical, the handedness of the user is determined to be left. The user is then prompted to confirm that they are left handed via the user interface and touchscreen 3.

By means of wireless communication with environmental identification devices that function as beacons identifying a location, e.g., an ambulance or hospital admission area, the RRCP Device 15 can be made intelligent enough to alter its state and its user interface based on its location. For example, when the gurney bearing the patient and the Defibrillator 16 arrive at the ambulance after the patient has been transported from the the point of the medical incident such as a myocardial infaction, cardiac arrest, or trauma, the Ambulance Identifier Beacon 19 transmits the identity of the vehicle to both the RRCP device 15 and the Defibrillator 16. The Ambulance Identifier Beacon 19 may be a simple 900 MHz fixed-data code transponder located on the roof of the ambulance, or may be a more sophisticated bidirectional device employing Bluetooth or other wireless communication technology. When the ambulance arrives at the hospital emergency department admission area, the RRCP Device 15 detects the Emergency Department Identifier Beacon 20 which will result in the RRCP Device automatically collecting all data from the Defibrillator 16 and any additional medical equipment used during the ambulance transport such as ventilators 21, chest compressors 23, or physiological monitors 22 can begin formatting the patient record in preparation for data record transmission or transfer and prompt the operator as a reminder that the record needs to be transferred.

As shown in FIG. 5, the Automatic Historical Preference Driven (AHPD) Menu display 30 of the user interface is varied depending on the type of treatment 31 currently being administered by the external medical device. This is determined from the state of the external medical device 17, e.g., monitoring, defibrillating, or pacing, via a communication link (II). The AHPD Menu 30 user interface is adjusted accordingly, with preference to the types of documentation done for the given type of treatment.

In preferred implementations, the user interface presents "situation aware" menu choices. There is a learning engine which keeps distinct databases 24 based on preferences shown by historical use for each type of treatment mode. The correct database is selected to drive the menu display, and updated based on choices made while this type of treatment is in progress.

Referring to FIG. 5, the Statistical Preference Database 24 can employ simple statistical methods such as a ranked-frequency listing of most recent choices from the list 1 or, preferably use more sophisticated and accurate methods for predicting the next item on the list 1 that the user will desire to enter. In the preferred embodiment, statistical learning methods are used such as Bayesian estimators, Kalman or particle filters or Markov models. In particular, the sequence of medical interventions is modelled as a hidden Markov model (HMM), defined as a variant of a finite state machine having a set of states, Q, an output alphabet, O, transition probabilities, A, output probabilities, B, and initial state probabilities, $\Pi$. The current state is not observable. Instead, each state produces an output with a certain probability (B). Usually the states, Q, and outputs, O, are understood, so an HMM is said to be a triple, $\lambda = (A, B, \Pi)$.

- A = $\{a_{ij} = P(q_j$ at t+1 $\mid q_i$ at t)$\}$, where $P(a \mid b)$ is the conditional probability of a given b, $t \geq 1$ is time, and $q_i \square$ Q. Informally, A is the probability that the next state is $q_j$ given that the current state is $q_i$.
- B = $\{b_{ik} = P(o_k \mid q_i)\}$, where $o_k \square$ O. Informally, B is the probability that the output is $o_k$ given that the current state is $q_i$.
- $\Pi = \{p_i = P(q_i$ at t=1)$\}$.

The Forward-Backward and Baum-Welch algorithms are performed on the database 24 to build the HMM. When a user first begins to use the RRPC device 15, the database will be small so in order to provide better predictive accuacy, a default HMM is used based on analysis of an aggregate of users collected previously. A global HMM is developed for all medical modes along with specific HMMs for each mode such as pacing, defibrillation, etc.

The Forward-Backward algorithm is summarized as follows:

Define the $\alpha$ values as follows,

$$\alpha\_t(i) = \Pr(O\_1 = o\_1, ..., O\_t = o\_t, X\_t = q\_i \mid \lambda)$$

Note that

$$\alpha\_T(i) = \Pr(O\_1 = o\_1, ..., O\_T = o\_T, X\_T = q\_i \mid \lambda)$$
$$= \Pr(\sigma, X\_T = q\_i \mid \lambda)$$

The alpha values enable us to solve Problem 1 since, marginalizing, we obtain

$$\Pr(\sigma \mid \lambda) = \text{sum}\_i = 1\textasciicircum N \ \Pr(o\_1, ..., o\_T, X\_T = q\_i \mid \lambda)$$
$$= \text{sum}\_i = 1\textasciicircum N \ \alpha\_T(i)$$

Define the $\beta$ values as follows,

$$\beta\_t(i) = \Pr(O\_t+1=o\_t+1,...,O\_T=o\_T \mid X\_t = q\_i, \lambda)$$

1. Compute the forward ($\alpha$) values:

    a.

$$\text{a. } \alpha\_1(i) = pi\_i \, b\_i(o\_1)$$

    b.

$$\text{b. } \alpha\_t+1(j) = [sum\_i=1^\wedge N \; \alpha\_t(i) \, a\_ij] \, b\_j(o\_t+1)$$

2. Computing the backward ($\beta$) values:

    a.

$$\text{a. } \beta\_T(i) = 1$$

    b.

$$\text{b. } \beta\_t(i) = sum\_j=1^\wedge N \; a\_ij \, b\_j(o\_t+1) \, \beta\_t+1(j)$$

The Baum-Welch algorithm is summarized as follows:

The probability of a trajectory being in state q_i at time t and making the transition to q_j at t+1 given the observation sequence and model.

$$xi\_t(i,j) = \Pr(X\_t = q\_i, X\_t+1 = q\_j \mid \sigma, \lambda)$$

We compute these probabilities using the forward backward variables.

$$xi\_t(i,j) = \frac{\alpha\_t(i) \, a\_ij(o\_t+1) \, \beta\_t+1(j)}{\Pr(O \mid \lambda)}$$

The probability of being in q_i at t given the observation sequence and model.
gamma_t(i) = Pr(X_t = q_i | $\sigma$, $\lambda$)
Which we obtain by marginalization.

$$\gamma\_t(i) = sum\_j \; xi\_t(i,j)$$

Note that
sum_t=1^T $\gamma$_t(i) = expected number of transitions from q_i and
sum_t=1^T xi_t(i,j) = expected number of transitions from q_i to q_j
Algorithm:

1. Choose the initial parameters, $\lambda$, arbitrarily.
2. Reestimate the parameters.

a.

$$\text{a. } \bar{\{\pi\}}\_i = \gamma\_t(i)$$

b.

$$\text{b. } \bar{\{a\}}\_ij = \frac{\text{sum\_t=1}^{\wedge}\text{T-1 xi\_t(i,j)}}{\text{sum\_t=1}^{\wedge}\text{T-1 } \gamma\_t(i)}$$

c.

$$\text{c. } \bar{\{b\}}\_j(k) = \frac{\text{sum\_t=1}^{\wedge}\text{T-1 } \gamma\_t(j) \ 1\_\{o\_t = k\}}{\text{sum\_t=1}^{\wedge}\text{T-1 } \gamma\_t(j)}$$

where 1_{o_t = k} = 1 if o_t = k and 0 otherwise.

3. Let bar {A} = {bar {a} _ ij} , bar {B} = {bar {b} _ i (k) } , and bar {π} = {{bar {π} _ i} .

4. Set bar{λ} to be {bar{A}, bar{B}, bar{π}}.

5. If λ= bar{λ} then quit, else set λto be bar{λ} and return to Step 2.

Based on the state transition probabilities calculated by the Baum-Welch algorithm, the Viterbi algorithm is used to provide a best estimate of the future sequence of medical interventions that the user will input.

The algorithm is summarized as follows:

1. Initialization:

For 1 <= i <= N,

a.

$$\text{a. } \delta\_1(i) = \pi b\_i(o\_1)$$

b.

$$\text{b. } \varphi\_1(i) = 0$$

2. Recursion:

For 2 <= t <= T, 1 <= j <= N,

a.

$$\text{a. } \delta\_t(j) = \max\_i \ [\delta\_t\text{-1}(i)a\_ij]b\_j(o\_t)$$

b.

$$\text{b. } \bar{\varphi}\_t(j) = \text{argmax}\_i \ [\delta\_t\text{-1}(i)a\_ij]$$

3. Termination:

   a.

$$a. \; p^* = \max\_i \; [\delta\_T(i)]$$

   b.

$$b. \; i^*\_T = \mathrm{argmax\_i} \; [\delta\_T(i)]$$

4. Reconstruction:

   For t = t- 1, t- 2, ..., 1,

$$i^*\_t = \phi\_t{+}1(i^*\_t{+}1)$$

The resulting trajectory, $i^*\_1$,..., $i^*\_t{+}1$, predicts the next likely intervention, based on the previous sequence.

The data created by the electronic RRPCs are analyzed by personnel responsible for quality control, such as the Medical Director. These computer-based analyses and reporting programs typically have user interfaces unique to that product, and the medical supervisory personnel are often burdened with the difficulty of learning new software functionality when new versions or products are available, and at times required to maintain skills in multiple complex analysis programs. In some implementations, the data environment in which the analysis is occurring is detected and the user interface of the analysis software takes on the appearance and operation of the software program that would normally be running to view that data file (e.g., if a data file from a Brand A defibrillator were received, the user interface of the analysis software would be configured to have the appearance of viewing software provided by Brand A).

Finally, when data is transferred to a desktop computer for review, the appearance of those tools is dynamically adjusted so that it is familiar in the original user context. For example, if data is recorded on a ZOLL M- Series CCT device, the data may be shown on the desktop in substantially the same form as it appeared originally on the portable device (e.g., a color screen, with the same number of boxes along the top of the screen, the same textual fonts, etc.) . In this way the desktop tools provide familiarity to a user trained on the portable device.

Aspects of the invention may include any of the following.

1. A portable electronic device for recording medical data, comprising
a display;
electronics for displaying a user interface on the display, and for responding to user inputs entered on the device, electronics for determining the environment in which the device is being used; wherein the user interface is varied in accordance with the determined environment.

2. The portable device of aspect 1 wherein the display is touch sensitive, and user inputs are made by touching selected portions of the display.

3. The portable device of aspect 2 wherein the user interface comprises buttons, and the choice of buttons displayed at a given time is varied depending on the environment in which the portable device is used.

4. The portable device of aspect 2 wherein the user interface comprises buttons and the position of the buttons at a given time is varied depending on the environment in which the portable device is used.

5. The portable device of aspect 1 wherein the user interface presents lists of items from which the user makes a selection, and a list presented at a given time contains a subset of items, wherein the content of the subset is varied in accordance with the environment in which the portable device is used.

6. The portable device of aspect 1 wherein the user interface is varied depending on detection of whether the user is holding the portable device with the left or the right hand.

7. The portable device of aspect 6 further comprising a sensor and related electronics for automatically detecting whether the portable device is being held along its right or left edge.

8. The portable device of aspect 1 further comprising electronics for communication with external devices, and the user interface is varied depending on the type of external device with which the portable device is communicating.

9. The portable device of aspect 1 further comprising electronics for communication and the mode in which the external device is operating is communicated to the portable device, and the user interface is varied depending on the mode in which the external device is operating.

10. The portable device of aspect 1 further comprising electronics for communication with an external device, and the user interface is varied in accordance with whether the portable device is in the vicinity of an external device.

11. The portable device of aspect 10 wherein the external device is a transmitter for informing the portable device that it is in the vicinity of the emergency room of a hospital.

12. The portable device of aspect 10 wherein the external device is a transmitter for informing the portable device that it is in an ambulance.

13. The portable device of aspect 8 wherein the user interface is varied depending on measurements made by the external device and communicated to the portable device.

14. The portable device of aspect 8 wherein the communication with an external device is in the form of wireless communication.

15. The portable device of aspect 1 wherein the user interface is varied depending on the medical interventions entered by the user.

16. The portable device of aspect 1 wherein the user interface is varied depending on the frequency with which actions have been taken in the past by the user.

17. The portable device of aspect 16 wherein the user interface is varied to reduce the number of steps necessary to select actions frequently taken in the past by the user.

18. The portable device of aspect 17 wherein a frequently selected action appears at the top of a list in the user interface.

19. The portable device of aspect 15 wherein the user interface is varied based on a predictive algorithm.

20. The portable device of aspect 19 wherein the predictive algorithm comprises one or more statistical learning methods.

21. The portable device of aspect 19 wherein the predictive algorithm comprises a ranked-frequency listing of recent choices from a list.

**Claims**

1. A portable electronic device for recording medical data, comprising
   a display;
   electronics for displaying a user interface on the display, and for responding to user inputs entered on the device,
   a sensor for determining proximity of the device to a location;
   electronics for processing the output of the sensor to determine the location in which the device is being used;
   wherein the user interface is varied in accordance with the determined location.

2. The portable device of claim 1 wherein the sensor is configured to determine proximity to a wireless transmitter positioned at the location.

3. The portable device of claim 2 wherein the wireless transmitter identifies at least one of an ambulance and an emergency department admissions area.

4. The portable device of claim 3 wherein, upon determining that the device is in the vicinity of an emergency department admissions area, the user interface provides a prompt to the operator relating to transfer of a patient record.

5. The portable device of claim 1 wherein the user interface is automatically varied in accordance with the determined location without any user interaction.

6. The portable device of claim 1 wherein the user interface comprises a plurality of user interface elements, and wherein the elements are varied with the result that the choices presented to the user, or the positions on the display of the choices, are varied.

7. The portable device of claim 6 wherein the display is touch sensitive, the user interface elements comprise selected touch-sensitive portions of the display, and user inputs are made by touching the selected touch-sensitive portions of the display.

8. The portable device of claim 7 wherein the user interface elements comprise buttons, and the choice of buttons displayed at a given time is varied depending on whether the device is being held with the right or left hand.

9. The portable device of claim 7 wherein the user interface elements comprise buttons and the position of the buttons at a given time is varied depending on whether the device is being held with the right or left hand.

10. The portable device of claim 1 wherein the user interface elements comprise items in one or more lists of items from which the user makes a selection, and a list presented at a given time contains a subset of items, wherein the content of the subset is varied in accordance with the location in which the portable device is used.

Figure 1

FIG. 1

Figure 2

Epinephrine, 1 mg, IV Push
Atropine, 1 mg, IV Push
Amiodarone, 300 mg, IV Push
Vasopressin, 40 Units, IV Push
IV Fluids...
Other...

10/03/2003 08:18 : Start of code log

Any UI Layer

24

Machine State Data    INPUTS    Z-Talk Packet

25

CRL
Command Request Layer

26

Only valid
command are
processed
unconditionally

OUTPUTS

27

Authoritative Command
Processor

**FIG. 3**

FIG. 4

I. Medical Device Treatment Mode _

II. CodeLink

IV. Statistical Preference Database

III. Current Treatment Type

V. Automatic Historical Preference Driven (AHPD) Menu Display

**FIG. 5**

FIGURE 6

Europäisches
Patentamt

European
Patent Office

Office européen
des brevets

# EUROPEAN SEARCH REPORT

Application Number

EP 13 16 8616

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 544 173 B2 (WEST KENNETH G [US] ET AL) 8 April 2003 (2003-04-08)<br>* figures 1-26 *<br>* column 1, line 43 - column 3, line 19 *<br>* column 11, line 16 - column 16, line 29 *<br>* column 24, line 62 - column 25, line 60 *<br>* column 28, line 34 - column 29, line 37 * | 1-10 | INV.<br>G06F19/00 |
| X | EP 1 286 247 A2 (HEWLETT PACKARD CO [US]) 26 February 2003 (2003-02-26)<br>* the whole document * | 1 | |
| A | US 2002/169584 A1 (FU ZHONGSU [US] ET AL) 14 November 2002 (2002-11-14)<br>* the whole document * | 1-10 | |
| A | HARRISON B L ET AL: "SQUEEZE ME, HOLD ME, TILT MEÜ AN EXPLORATION OF MANIPULATIVE USER INTERFACES",<br>CHI '98. HUMAN FACTORS IN COMPUTING SYSTEMS. CONFERENCE PROCEEDINGS. LOS ANGELES, CA, APRIL 18 - 23, 1998; [CHI CONFERENCE PROCEEDINGS. HUMAN FACTORS IN COMPUTING SYSTEMS], NEW YORK, NY : ACM, US,<br>18 April 1998 (1998-04-18), pages 17-24, XP000780770,<br>ISBN: 978-0-89791-975-3<br>* page 18, right-hand column, line 8 - page 18, right-hand column, line 32 *<br>* page 20, left-hand column, line 18 - page 21, left-hand column, line 10 * | 8,9 | TECHNICAL FIELDS SEARCHED (IPC)<br><br>G06F |

-/--

The present search report has been drawn up for all claims

| Place of search | Date of completion of the search | Examiner |
|---|---|---|
| The Hague | 9 August 2013 | Hernández Marugán, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 13 16 8616

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2003/103038 A1 (WONG YOON KEAN [US] ET AL) 5 June 2003 (2003-06-05) <br> * paragraph [0036] - paragraph [0038] * <br> * paragraph [0045] - paragraph [0068] * <br> * figures 1-7 * <br> ----- | 8,9 | |

TECHNICAL FIELDS
SEARCHED        (IPC)

The present search report has been drawn up for all claims

| Place of search | Date of completion of the search | Examiner |
|---|---|---|
| The Hague | 9 August 2013 | Hernández Marugán, J |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&  : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 13 16 8616

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2013

| Patent document cited in search report | | Publication date | Patent family member(s) | | Publication date |
|---|---|---|---|---|---|
| US 6544173 | B2 | 08-04-2003 | AT | 502567 T | 15-04-2011 |
| | | | AU | 6465401 A | 03-12-2001 |
| | | | AU | 2001264654 B2 | 16-06-2005 |
| | | | EP | 1404213 A2 | 07-04-2004 |
| | | | ES | 2362414 T3 | 05-07-2011 |
| | | | US | 6616606 B1 | 09-09-2003 |
| | | | US | 2002013517 A1 | 31-01-2002 |
| | | | US | 2002013518 A1 | 31-01-2002 |
| | | | US | 2003206116 A1 | 06-11-2003 |
| | | | US | 2006030759 A1 | 09-02-2006 |
| | | | WO | 0189362 A2 | 29-11-2001 |
| EP 1286247 | A2 | 26-02-2003 | EP | 1286247 A2 | 26-02-2003 |
| | | | US | 6505121 B1 | 07-01-2003 |
| US 2002169584 | A1 | 14-11-2002 | NONE | | |
| US 2003103038 | A1 | 05-06-2003 | US | 2003103038 A1 | 05-06-2003 |
| | | | US | 2005184955 A1 | 25-08-2005 |
| | | | US | 2007296693 A1 | 27-12-2007 |
| | | | US | 2010171699 A1 | 08-07-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 51290803 P